# FASCICULE DE BREVET EUROPEEN

(11) **EP 3 416 708 B1**
(45) Date de publication et mention de la délivrance du brevet: **10.11.2021**
(21) Numéro de dépôt: 17708863.0
(22) Date de dépôt: 13.02.2017
(51) Int. Cl.: A61M 5/20, A61M 5/30, A61M 5/315, A61M 5/28

(54) **DISPOSITIF D'INJECTION SANS AIGUILLE A MEMBRANE COULISSANTE**
NADELLOSE INJEKTORVORRICHTUNG MIT GLEITMEMBRAN
NEEDLE-LESS INJECTOR DEVICE HAVING A SLIDING MEMBRANE

(30) Priorité: 18.02.2016 FR 1651340
(43) Date de publication de la demande: 26.12.2018
(73) Titulaire: Crossject, 21000 Dijon (FR)
(72) Inventeur: VIGOT, Xavier, 21260 Veronnes (FR); AURIEL, Christophe, 21270 Binges (FR)
(74) Mandataire: Germain Maureau
(86) Numéro de dépôt international: PCT/FR2017/050322
(87) Numéro de publication internationale: WO 2017/140973

(56) Documents cités:
- CH-A5- 580 427
- FR-A1- 2 852 516

## Description

L'invention concerne un dispositif d'injection sans aiguille comprenant une membrane dont le coulissement dans le réservoir est favorisé.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans un logement tubulaire délimité par le corps du dispositif, le tube étant obturé par un piston amont et un piston aval entre lesquels est contenu le principe actif liquide.

L'extrémité libre aval, ou inférieure, du réservoir, coopère avec une buse d'injection qui délimite au moins un canal d'injection s'étendant axialement suivant un axe d'injection.

La buse d'injection est délimitée axialement par une face supérieure en appui axial sur le réservoir, et une face inférieure d'injection adaptée pour coopérer avec un opercule de fermeture.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appui la buse d'injection sur sa peau.

Le déplacement du corps dans le capot permet le déclenchement du générateur de gaz, générant un gaz sous pression qui entraîne en déplacement les pistons pour injecter le principe actif à travers la peau du patient, en passant par la buse d'injection.

On connait un dispositif d'injection qui est équipé d'une membrane déformable élastiquement de forme globalement en T, qui comprend un disque annulaire radial qui est interposé axialement entre l'extrémité supérieure du réservoir et un siège formé par le corps, et une partie tubulaire qui s'étend axialement dans le réservoir, depuis le disque annulaire.

La partie tubulaire de la membrane est conçue pour s'étendre axialement sous l'effet du gaz sous pression, afin d'entraîner les pistons en déplacement.

La pression du gaz déforme également la membrane radialement, de sorte que le membrane vient en contact sur la paroi interne du réservoir en verre.

Le frottement entre la membrane, qui est généralement réalisée en élastomère, et la paroi en verre du réservoir, est important et absorbe une partie importante de l'énergie nécessaire à l'allongement et l'extension de la partie tubulaire de la membrane dans le réservoir.

Pour pallier ce problème, il est connu de lubrifier la membrane pour limiter le frottement entre la membrane et le réservoir.

Bien qu'efficace, la lubrification est une étape contraignante au cours de la réalisation et de l'assemblage du dispositif d'injection.

D'autres dispositifs d'injection connus sont décrits dans les documents FR2852516 et CH580427.

La présente invention est définie par la revendication 1 et vise notamment à résoudre cet inconvénient. La présente invention se rapporte pour ce faire à un dispositif d'injection sans aiguille comportant :
- un corps formant un logement,
- un générateur de gaz,
- un réservoir tubulaire qui contient un principe actif à injecter, le réservoir s'étendant axialement dans ledit logement depuis une extrémité supérieure, jusqu'à une extrémité inférieure,
- une membrane déformable élastiquement de forme globalement en T, la membrane comprenant une partie tubulaire qui s'étend axialement dans le réservoir et qui est conçue pour s'allonger axialement dans le réservoir sous l'effet de la pression générée par le générateur de gaz, et
- une buse d'injection du principe actif qui est agencée à l'extrémité inférieure du réservoir, ledit dispositif étant caractérisé en ce qu'il comporte une chaussette qui enveloppe la partie tubulaire de la membrane au moins en partie, la chaussette étant adaptée pour limiter le frottement entre la partie tubulaire de la membrane et le réservoir.

L'invention permet de limiter l'énergie absorbée par le frottement entre la membrane et le réservoir, afin d'obtenir une pression de jet à travers la buse satisfaisante.

On constate que la chaussette améliore la puissance du jet de principe actif en sortie de buse.

La chaussette favorise également le déploiement et l'allongement de la membrane dans le réservoir.

Selon une autre caractéristique, la chaussette présente la forme d'une douille qui comprend :
- une paroi cylindrique qui s'étend axialement et qui est interposée radialement entre une paroi interne du réservoir et la partie tubulaire de la membrane, et
- un fond qui obstrue la chaussette.

La forme cylindrique de la chaussette, complémentaire à la forme du réservoir, favorise le glissement de la douille dans le réservoir.

Selon une autre caractéristique, pour minimiser les frottements, la chaussette est réalisée dans un matériau qui présente un coefficient de frottement inférieur au coefficient de frottement du matériau utilisé pour réaliser la membrane.

De préférence, la membrane est réalisée en matériau à base d'élastomère.

Selon un exemple de réalisation, la chaussette est en plastique.

Selon un exemple de réalisation de l'invention, la chaussette est suffisamment rigide pour ne pas se déformer radialement sous l'effet de la pression générée par le générateur de gaz.

Cette caractéristique vise à limiter la surface de contact entre la chaussette et le réservoir, afin de limiter le frottement.

Selon un exemple de réalisation de l'invention, la membrane comprend un disque annulaire radial qui est relié sur la partie tubulaire de la membrane, le disque annulaire étant en appui axial sur une extrémité supérieure du réservoir.

Selon une autre caractéristique, la chaussette est montée coulissante dans le réservoir et présente un diamètre sensiblement inférieur au diamètre de la paroi interne du réservoir.

Selon une autre caractéristique, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en section axiale qui illustre un dispositif d'injection comportant une chaussette enveloppant la membrane en position de repos, selon l'invention ;
- la figure 2 est une vue en section axiale qui illustre le dispositif d'injection de la figure 1 avec la membrane en position allongée ;
- la figure 3 est une vue de détail en section axiale illustrant la membrane et la chaussette de la figure 1 ;
- la figure 4 est une vue de détail en perspective illustrant la chaussette cylindrique de la figure 1.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe B d'injection du dispositif d'injection.

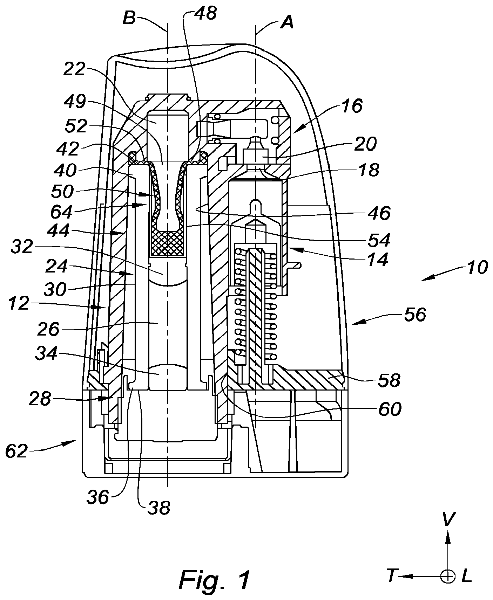

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston amont 32 et un piston aval 34 entre lesquels est contenu le principe actif 26 liquide, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

Le réservoir 24 est agencé dans un logement 44 formé par le corps 12, logement 44 qui est délimité radialement par une paroi 46 tubulaire qui s'étend autour de l'axe B d'injection.

Le logement 44 s'étend axialement depuis un siège 48 radial supérieur qui est formé par le corps 12 et qui délimite un orifice de sortie 49 de la chambre d'expansion 22.

Selon un exemple de réalisation préféré, le corps 12 est réalisé par moulage en injection de matière plastique.

Aussi, selon la figure 1, le dispositif 10 est équipé d'une membrane 50 déformable élastiquement de forme globalement en T, qui comprend un disque 52 annulaire radial qui est interposé axialement entre la collerette supérieure 40 du réservoir 24 et le siège 48 formé par le corps 12, et une partie tubulaire 54 qui s'étend axialement dans le réservoir 24, depuis le disque 52 annulaire.

La partie tubulaire 54 de la membrane 50 est conçue pour s'étendre axialement, sous l'effet de la pression du gaz généré par le générateur de gaz 16, pour pousser le piston amont 32 vers le bas afin d'éjecter le principe actif 26 à travers la buse 28 d'injection.

A cet effet, la membrane 50 est réalisée dans un matériau à base d'élastomère.

En référence à la figure 1, le corps 12 est enveloppé par un capot 56 creux qui délimite une ouverture inférieure fermée par une semelle 58 horizontale formant fond de capot.

La semelle 58 délimite un passage circulaire 60 autour de l'axe B d'injection qui est adapté pour le passage de la buse 28 d'injection et de l'extrémité aval du corps 12, de sorte que la buse 28 comporte un tronçon inférieur faisant saillie verticalement vers le bas hors du capot 56.

Plus particulièrement, la buse 28 est vissée sur une extrémité libre débouchante du logement 44 formé par le corps 12, la buse 28 comprimant axialement l'ensemble formé par le réservoir 24 et la membrane 50 sur le siège 48 du logement 44.

Aussi, le dispositif 10 d'injection est équipé d'un bouchon 62 qui est monté sur le corps 12 de façon amovible par un moyen de verrouillage du type à baïonnette.

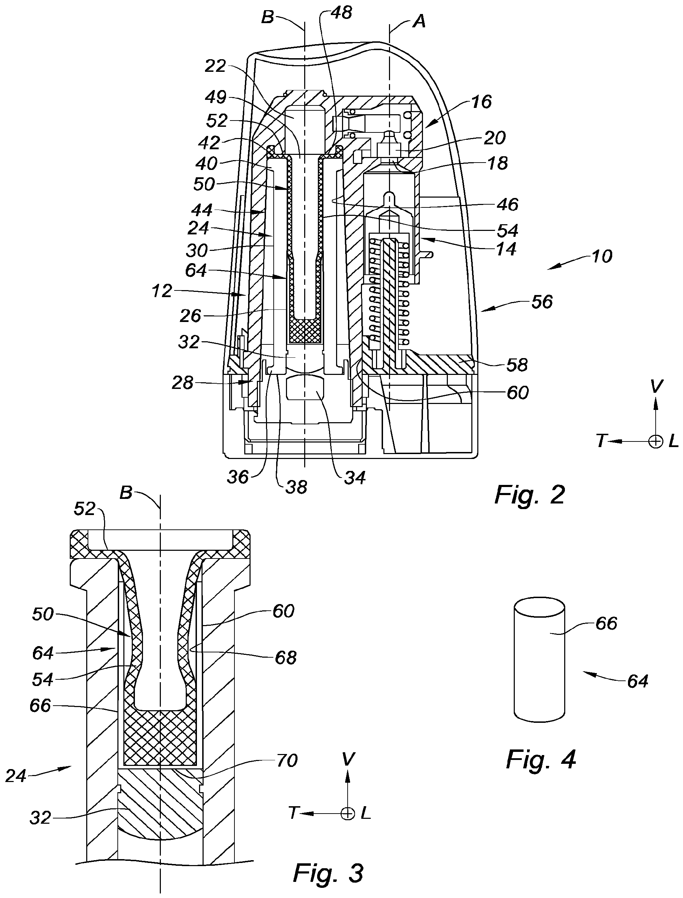

Conformément à l'invention, le dispositif 10 comporte une chaussette 64, représentée en détail à la figure 4, qui enveloppe la partie tubulaire 54 de la membrane 50.

Comme on peut le voir à la figure 3, la chaussette 64 présente la forme d'une douille qui comprend une paroi cylindrique 66 qui s'étend axialement suivant l'axe B d'injection et qui est interposée radialement entre la paroi interne 68 du réservoir 24 et la partie tubulaire 54 de la membrane 50.

Aussi, la chaussette 64 comporte un fond 70 en forme de disque qui obstrue axialement la chaussette 64 dans sa partie inférieure.

Le fond 70 de la chaussette 64 peut présenter une épaisseur verticales qui varie en fonction du volume du principe actif 26 contenu dans le réservoir 24. Ainsi, plus le volume de principe actif est réduit, plus l'épaisseur du fond 70 de la chaussette 64 sera épais, la surépaisseur de la chaussette compensant le volume de principe actif.

La chaussette 64 est réalisée dans un matériau qui présente un coefficient de frottement inférieur au coefficient de frottement du matériau utilisé pour réaliser la membrane 50, pour permettre à la chaussette 64 de limiter le frottement entre la partie tubulaire 54 de la membrane 50 et le réservoir 24.

Selon un exemple de réalisation, la chaussette 64 est réalisée en plastique lisse.

A titre non limitatif, la chaussette peut également être réalisée en aluminium ou tout autre matériau présentant un faible coefficient de frottement sur le verre.

Lorsque le générateur de gaz 16 est activé, le gaz sous pression pénètre dans la membrane 50 et la partie tubulaire 54 de la membrane 50 s'étend axialement vers le bas sous l'effet de la pression générée par le gaz, et simultanément, la chaussette 64 est entraînée en coulissement axial vers la bas avec la partie tubulaire 54 de la membrane 50.

Ainsi, la chaussette 64, grâce à son coefficient de frottement réduit, glisse sur la paroi interne 68 du réservoir 24 pour favoriser l'allongement axial de la membrane 50.

Selon un autre aspect de l'invention, la chaussette 64 est suffisamment rigide pour ne pas, ou peu, se déformer radialement sous l'effet de la pression générée par le générateur de gaz 16, afin de limiter le contact et le frottement entre la chaussette 64 et le réservoir 24.

Dans ce but, la chaussette 64 peut être réalisée en aluminium par exemple.

A titre d'exemple de réalisation, une chaussette 64 en aluminium présente un diamètre intérieur de 6 millimètres et un diamètre extérieur de 6,8 millimètres pour être rigide. Aussi, une chaussette 64 en polyamide fibré présente un diamètre intérieur de 5,4 millimètres et un diamètre extérieur de 6,8 millimètres pour être rigide

De façon complémentaire, la chaussette 64 est montée coulissante dans le réservoir 24 et elle présente un diamètre sensiblement inférieur au diamètre de la paroi interne 68 du réservoir 24.

Ces caractéristiques permettent de limiter la surface de contact, et donc le frottement, entre la chaussette 64 et le réservoir 24 au cours de l'allongement de la membrane 50.

Selon un autre aspect de l'invention, le principe actif 26 contenu dans le réservoir 24 est choisi parmi le groupe comprenant les principes actifs suivants : Methotrexate, Adrénaline, Sumatriptan, Hydrocortisone, Naloxone, Midazolam, Apomorphine, Ethylnatrexone bromide, Phytomenadione, Chlorpromazine hydrocloride, Zuclopenthixol acetate, Danaparoid sodium, Enoxaparin sodium, Estradiol cypionate, Medoxyprogesterone acetate, Medroparin calcium, Methylprednisolone acetate Heparin calcium, Terbuline.

La présente description de l'invention est donnée à titre d'exemple non limitatif.

On comprendra notamment que la chaussette 64 peut présenter une longueur égale ou supérieure à la longueur axiale de la partie tubulaire 54 de la membrane 50.

## Revendications

1. Dispositif (10) d'injection sans aiguille comportant :
- un corps (12) formant un logement (44),
- un générateur de gaz (16),
- un réservoir (24) tubulaire qui contient un principe actif (26) à injecter, le réservoir (24) s'étendant axialement dans ledit logement (44) depuis une extrémité supérieure, jusqu'à une extrémité inférieure (36),
- une membrane (50) déformable élastiquement de forme globalement en T, la membrane (50) comprenant une partie tubulaire (54) qui s'étend axialement dans le réservoir (24) et qui est conçue pour s'allonger axialement dans le réservoir (24) sous l'effet de la pression générée par le générateur de gaz (16), et
- une buse (38) d'injection du principe actif (26) qui est agencée à l'extrémité inférieure du réservoir (24),
ledit dispositif (10) étant **caractérisé en ce qu'**il comporte une chaussette (64) qui enveloppe la partie tubulaire (54) de la membrane (50) au moins en partie, ladite chaussette
- présentant la forme d'une douille, qui comprend une paroi cylindrique qui s'étend axialement et qui est interposée radialement entre une paroi interne du réservoir et la partie tubulaire de la membrane la chaussette (64) étant réalisée dans un matériau qui présente un coefficient de frottement inférieur au coefficient de frottement du matériau utilisé pour réaliser la membrane (50) de sorte à limiter le frottement entre la partie tubulaire (54) de la membrane (50) et le réservoir (24),
- et comprenant un fond (70) en forme de disque qui obstrue axialement la chaussette (64) dans sa partie inférieure.

2. Dispositif (10) d'injection sans aiguille selon la revendication 1, **caractérisé en ce que** la membrane (50) est réalisée en matériau à base d'élastomère.

3. Dispositif (10) d'injection sans aiguille selon la revendication 1 ou 2, **caractérisé en ce que** la chaussette (64) est en plastique.

4. Dispositif (10) d'injection sans aiguille selon l'une quelconque des revendications précédentes, **caractérisé en ce que** la chaussette (64) est suffisamment rigide pour ne pas se déformer radialement sous l'effet de la pression générée par le générateur de gaz (16).

5. Dispositif (10) d'injection sans aiguille selon l'une quelconque des revendications précédentes, **caractérisé en ce que** la membrane (50) comprend un disque (52) annulaire radial qui est relié sur la partie tubulaire (54) de la membrane (50), le disque (52) annulaire étant en appui axial sur une extrémité supérieure du réservoir (24).

6. Dispositif (10) d'injection sans aiguille selon l'une quelconque des revendications précédentes, **caractérisé en ce que** la chaussette (64) est montée coulissante dans le réservoir (24) et présente un diamètre sensiblement inférieur au diamètre de la paroi interne (68) du réservoir (24).

7. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadellose Injektionsvorrichtung (10), aufweisend:
- einen Köper (12), der eine Aufnahme (44) bildet,
- einen Gaserzeuger (16),
- einen rohrförmigen Vorratsbehälter (24), der einen zu injizierenden Wirkstoff (26) enthält, wobei sich der Vorratsbehälter (24) in der Aufnahme (44) ab einem oberen Ende bis zu einem unteren Ende (36) axial erstreckt,
- eine elastisch verformbare Membran (50) in allgemeiner T-Form, wobei die Membran (50) einen rohrförmigen Teil (54) umfasst, der sich im Vorratsbehälter (24) axial erstreckt und der ausgebildet ist, um sich im Vorratsbehälter (24) unter der Wirkung des von dem Gaserzeuger (16) erzeugten Drucks axial zu strecken, und
- eine Injektionsdüse (38) des Wirkstoffs (26), die am unteren Ende des Vorratsbehälters (24) eingerichtet ist,
wobei die Vorrichtung (10) **dadurch gekennzeichnet ist, dass** sie einen Strumpf (64) aufweist, der den rohrförmigen Teil (54) der Membran (50) mindestens zum Teil umgibt, wobei der Strumpf
- die Form einer Hülse aufweist, die eine zylindrische Wand umfasst, die sich axial erstreckt und die zwischen einer Innenwand des Vorratsbehälters und dem rohrförmigen Teil der Membran radial angeordnet ist, wobei der Strumpf (64) aus einem Material hergestellt ist, das einen Reibungskoeffizienten aufweist, der niedriger als der Reibungskoeffizient des Materials ist, das für die Herstellung der Membran (50) verwendet wird, so dass die Reibung zwischen dem rohrförmigen Teil (54) der Membran (50) und dem Vorratsbehälter (24) begrenzt wird,
- und einen scheibenförmigen Boden (70) umfasst, der den Strumpf (64) in seinem unteren Teil axial verschließt.

2. Nadellose Injektionsvorrichtung (10) nach Anspruch 1, **dadurch gekennzeichnet, dass** die Membran (50) aus Material auf der Basis von Elastomer hergestellt ist.

3. Nadellose Injektionsvorrichtung (10) nach Anspruch 1 oder 2, **dadurch gekennzeichnet, dass** der Strumpf (64) aus Kunststoff ist.

4. Nadellose Injektionsvorrichtung (10) nach einem der vorangehenden Ansprüche, **dadurch gekennzeichnet, dass** der Strumpf (64) ausreichend steif ist, um sich unter dem von dem Gaserzeuger (16) erzeugten Druck radial nicht zu verformen.

5. Nadellose Injektionsvorrichtung (10) nach einem der vorangehenden Ansprüche, **dadurch gekennzeichnet, dass** die Membran (50) eine radiale ringförmige Scheibe (52) umfasst, die auf dem rohrförmigen Teil (54) der Membran (50) verbunden ist, wobei sich die ringförmige Scheibe (52) auf einem oberen Ende des Vorratsbehälters (24) axial abstützt.

6. Nadellose Injektionsvorrichtung (10) nach einem der vorangehenden Ansprüche, **dadurch gekennzeichnet, dass** der Strumpf (64) in dem Vorratsbehälter (24) gleitend angebracht ist und einen Durchmesser aufweist, der etwas kleiner als der Durchmesser der Innenwand (68) des Vorratsbehälters (24) ist.

7. Nadellose Injektionsvorrichtung (10) nach einem der vorangehenden Ansprüche, **dadurch gekennzeichnet, dass** der in dem Vorratsbehälter (24) enthaltene Wirkstoff (26) aus der Gruppe ausgewählt ist, die die folgenden Wirkstoffe umfasst:
- Methotrexat,
- Adrenalin,
- Sumatriptan,
- Hydrocortison,
- Naloxon,
- Midazolam,
- Apomorphin,
- Ethylnatrexon-Bromid,
- Phytomenadion,
- Chlorpromazin-Hydroclorid,
- Zuclopenthixolacetat,
- Danaparoid-Natrium,
- Enoxaparin-Natrium,
- Östradiolcypionat,
- Medoxyprogesteronacetat,
- Medroparin-Calcium,
- Methylprednisolonacetat,
- Heparin-Calcium,
- Terbulin.

## Claims

1. A needleless injection device (10) including:
- a body (12) forming a housing (44),
- a gas generator (16),
- a tubular reservoir (24) which contains an active ingredient (26) to be injected, the reservoir (24) extending axially in said housing (44) from an upper end, down to a lower end (36),
- an elastically-deformable membrane (50) with a generally T-like shape, the membrane (50) comprising a tubular portion (54) which extends axially in the reservoir (24) and which is designed to extend axially in the reservoir (24) under the effect of the pressure generated by the gas generator (16), and
- a nozzle (38) for injecting the active ingredient (26) which is arranged at the lower end of the reservoir (24),
said device (10) being **characterized in that** it includes a sock (64) which envelops at least partly the tubular portion (54) of the membrane (50), said sock:
- being in the form of a sleeve, which comprises a cylindrical wall which extends axially and which is interposed radially between an inner wall of the reservoir and the tubular portion of the membrane, the sock (64) being made of a material that has a coefficient of friction lower than the coefficient of friction of the material used to make the membrane (50) so as to limit the friction between the tubular portion (54) of the membrane (50) and the reservoir (24),
- and comprising a bottom (70) in the form of a disc which axially plugs the sock (64) in its lower portion.

2. The needleless injection device (10) according to claim 1, **characterized in that** the membrane (50) is made of an elastomer-based material.

3. The needleless injection device (10) according to claim 1 or 2, **characterized in that** the sock (64) is made of plastic.

4. The needleless injection device (10) according to any one of the preceding claims, **characterized in that** the sock (64) is sufficiently rigid so as not to deform radially under the effect of the pressure generated by the gas generator (16).

5. The needleless injection device (10) according to any one of the preceding claims, **characterized in that** the membrane (50) comprises a radial annular disc (52) which is connected to the tubular portion (54) of the membrane (50), the annular disc (52) bearing axially on an upper end of the reservoir (24).

6. The needleless injection device (10) according to any one of the preceding claims, **characterized in that** the sock (64) is slidably mounted in the reservoir (24) and has a diameter substantially smaller than the diameter of the inner wall (68) of the reservoir (24).

7. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.
